# EUROPEAN PATENT APPLICATION

(11) **EP 2 389 970 A1**
(43) Date of publication of application: **30.11.2011**
(21) Application number: 10164431.8
(22) Date of filing: 31.05.2010
(51) Int. Cl.: A61M 16/00, A61M 16/20

(54) **Breathing circuit pressure control system**

(71) Applicant: General Electric Company, Schenectady, NY 12345 (US)
(72) Inventor: Parviainen, Olli, 02600, Espoo (FI)
(74) Representative: Valkeiskangas, Tapio Lassi Paavali

(57) **Abstract**

Breathing circuit pressure control system in connection of ventilation, controlling pressure in an airway line of the breathing circuit, the breathing circuit pressure control system comprising, a flow control element (9) for controlling flow resistance in the airway line (11), an electromagnetic actuator (10) for creating a force acting on the flow control element (9), and a ventilator control unit (21) to control the electromagnetic actuator for creating a force acting on the flow control element (9). The breathing circuit pressure control system further comprises an another electrical control system (12,14,16) to control the electromagnetic actuator for creating a force acting on the flow control element (9), and a selector switch (24) for selecting the another electrical control unit arranged to control the electromagnetic actuator and disconnect the ventilator control unit (21) from controlling the electromagnetic actuator (10).

## Description

### BACKGROUND OF THE INVENTION

The disclosure relates to a breathing circuit pressure control system in connection of ventilation controlling pressure in an airway line of the breathing circuit the breathing circuit pressure control system comprising, a flow control element for controlling flow resistance in the airway line, an electromagnetic actuator for creating a force acting on the flow control, and a ventilator control unit to control the electromagnetic actuator for creating a force acting on the flow control element.

Inhalation anaesthesia delivery machines provide means for both machine and manual ventilation of the patient during anaesthesia. Typical manual ventilation arrangement involves an elastic bag connected to patient airways through the anaesthesia delivery system breathing circuit, so that clinician squeezing the bag can manually increase the airway pressure and thus make gas flow from the breathing circuit to enter the patient airways.

While automatic machine ventilation is used for patient respiration for most of the time during the operation,
1) At beginning and end of the anaesthesia operation manual ventilation is used as a transition phase to and from machine ventilation state, and
2) The clinician may occasionally switch from machine to manual ventilation also during the case to check patient's condition or use manual ventilation as a fallback option in case of problems in machine ventilation delivery.

During manual ventilation an Airway Pressure Limit (APL) device is often used to regulate maximum airway pressure level. APL device is a pressure relief valve that opens at user-set pressure level to avoid excessive pressure levels at patient airways. Traditional APL valve construction is a mechanical spring-loaded valve that user can adjusts by turning a mechanical dial that affects the valve spring force.

Manual ventilation arrangement featuring a mechanical APL can operate without electric power and thus provide backup means for electronic machine ventilation failures, but from user's point of view APL usage has some setbacks such as
a) lack of sensitivity how much of the gas flows really to patient,
b) when frequent adjustment of APL is required, other of clinician's hand get occupied for that,
c) Mechanical APL valve involves more pieces subject to periodic cleaning and maintenance,
d) Mechanical APL is expensive, increasing system cost,
e) Mechanical APL limits anaesthesia machine design, because the APL has to reside in anaesthesia machine pneumatic circuit and have its own mechanical user interface there.

The disclosure presents an electric arrangement for APL operation that can avoid above limitations.

### BRIEF DESCRIPTION OF THE INVENTION

The shortcomings, disadvantages and problems of mechanical APL are addressed herein which will be understood by reading and understanding the following specification.

In an embodiment, breathing circuit pressure control system in connection of ventilation controlling pressure in an airway line of the breathing circuit the breathing circuit pressure control system comprising, a flow control element for controlling flow resistance in the airway line, an electromagnetic actuator for creating a force acting on the flow control element, and a ventilator control unit to control the electromagnetic actuator for creating a force acting on the flow control element. The breathing circuit pressure control system further comprises an another electrical control system to control the electromagnetic actuator for creating a force acting on the flow control element, and a selector switch for selecting the another electrical control system arranged to control the electromagnetic actuator and disconnect the ventilator control unit from controlling the electromagnetic actuator.

In another embodiment the system comprises a mechanical ventilation system and a manual ventilation system, and the another electrical control system has been arranged to be used when the manual ventilation system is used. It is also possible that the mechanical ventilation system comprises an anaesthesia delivery machine.

The embodiments described above present electric arrangement with back-up power using the ventilator exhalation valve to provide APL (Airway Pressure Limit) functionality comparable to traditional anaesthesia machine mechanical APL functionality to allow delivering manual ventilation, either as a primary APL mechanism for general anaesthesia device without need for separate mechanical APL control valve, or as a backup mechanism for novel electronically controlled manual ventilation in case of sudden machine ventilator or ventilator power supply failure.

### BRIEF DESCRIPTION OF THE DRAWINGS

Figure 1 shows schematically a typical ventilation arrangement enabling both mechanical and manual ventilations of a patient,
Figure 2 shows schematically a typical prior art exhalation valve arrangement based of a ventilator-controlled electromagnetic actuator, and
Figure 3 shows schematically an embodiment of the present invetion.

### DETAILED DESCRIPTION OF THE INVENTION

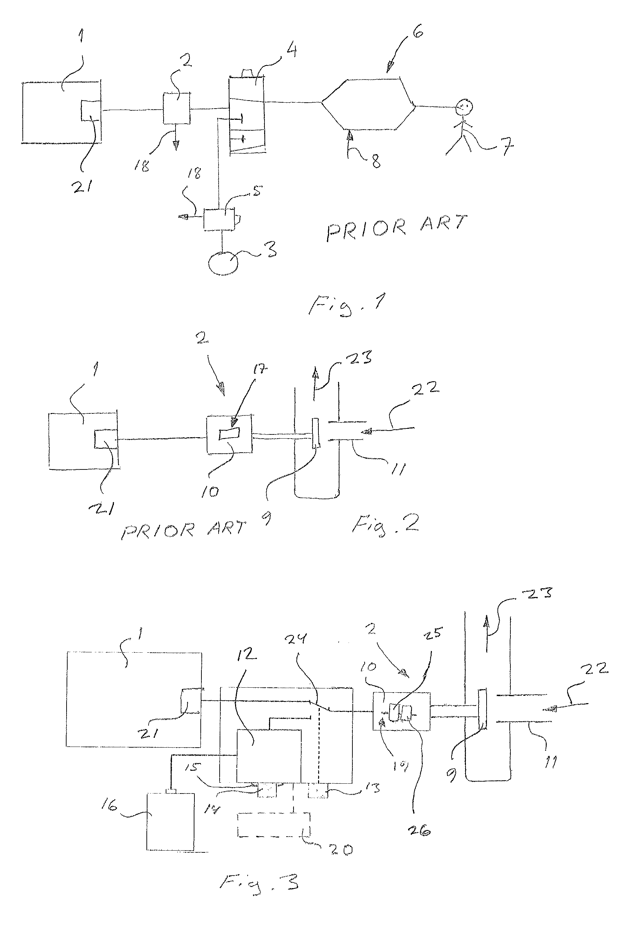

Figure 1 shows schematically a state-of-the art ventilation arrangement enabling both mechanical and manual ventilations of a patient. Reference number 1 shows a ventilator including a ventilator control unit 21. Reference number 2 shows an exhalation valve including a ventilator controlled actuator. Reference number 3 shows an elastic bag for manual ventilation and reference number 4 shows a manual/mechanical ventilation valve, i.e. a valve by which manual mode or machine operated ventilation can be selected. Reference number 5 shows a mechanical APL control valve which is used during manual ventilation to regulate maximum pressure level to avoid excessive pressure levels in patient airways.

Reference number 6 in Figure 1 shows a patient circuit and reference number 7 shows a patient. An arrow 8 shows schematically a flow of fresh gas to the patient circuit. Reference number 18 shows schematically gas that is scavenged out from the system.

Figure 1 must be understood here so that Figure 1 shows a basic operation principle of ventilation of a patient, i.e. mechanical ventilation and its control is carried out by a ventilator 1 and manual ventilation is carried out manually by using the elastic bag 3. Breathing circuit pressure during manual ventilation is controlled by the mechanical APL control valve as discussed above. It is important to understand here that two valves i.e. valve 2 is used in mechanical ventilation mode and valve 5 is used in manual ventilation mode. Manual ventilation with mechanical APL valve 5 provides also a backup means for electronic machine failures.

Referring generally to Figure 1 the term ventilator must be interpreted here widely, i.e. the ventilator may also comprise an anaesthesia delivery machine etc.

The matters, i.e. constructive details and operation in detail discussed above are well known to a person skilled in the art and therefore the details of the arrangement are not discussed in detail here.

Typically electronic ventilator controls patient airway pressure and expiration flow from patient's lungs by using an exhalation valve 2 consisting of a flow control element 9, for example a restrictor diaphragm, that is actuated e.g. by an electromagnetic actuator 10 such as a coil 17, i.e. a solenoid coil or voice coil as shown schematically in Figure 2. In Figure 2 reference number 11 shows a patient airway line, i.e. an exhalation line and the arrow 22 in Figure 2 shows the exhalation gas flow in the patient airway line 11. Ventilator is shown schematically in Figure 2 with reference number 1. The ventilator 1 includes a ventilator control unit 21 to control the electromagnetic actuator 10 in order to maintain desired pressure in the breathing circuit. For this control the ventilator control unit may utilize pressure sensors measuring the breathing circuit pressure.

As told above Figure 2 shows schematically an exhalation valve based on ventilator-controlled electromagnetic actuator. The electromagnetic actuator 10 exerts force to flow control element 9, for example the exhalation valve diaphragm, which moves to alternate flow resistance in the patient airway line 11 between freely flowing and fully closed position. Arrow 23 shows the exhalation flow after the flow control element 9.

In case of a ventilator control unit 21 failure, the coil 17 in the exhalation valve actuator may deactivate or freeze, resulting to either release of the patient airway line pressure or locking to fixed pressure limit level, depending on the failure mode. In ventilator that the exhalation valve is used as a device for maintaining or regulating the patient airway pressure level during manual ventilation, failure of ventilator control results in losing manual backup ventilation also. That is one of the reasons why mechanical APL valves are used as backup means for electronic machine ventilation failures as told earlier.

The new embodiment described in detail later introduces independent electric APL control apparatus that when activated, takes over control of the exhalation valve from the ventilator and feeds user-adjustable electric current directly into an exhalation valve actuator coil. User can control the actuator drive by suitable control apparatus, and as the user-controlled electric drive converts to corresponding force to the flow control element, the user can use this control apparatus as means of setting the airway pressure limit level in similar way as traditional mechanical APL valve. This leads to elimination of the mechanical APL valve but still offers a backup for technical failures on ventilator control unit. The new embodiment offers also possibility without a mechanical APL valve to use manual ventilation in safely manner. The arrangement becomes thus cheaper when compared to the arrangement of the prior art.

The new embodiment referred to above is shown schematically in Figure 3. Figure 3 shows an arrangement that introduces an alternative APL control mode capability. Figure 3 shows a similar electromagnetic actuator 10 and exhalation valve 2 as in Figure 2, but the embodiment of Figure 3 has an another, i.e. an additional electrical control 12, 14, 16 for the electromagnetic actuator 10 that allows controlling the exhalation flow and breathing circuit pressure in case that the ventilator control unit 21 fails.

When the another or additional electrical control system 12, 14, 16 is connected to control the actuator, for example by using a selector switch knob 13 connected to actuate a selector switch 24, the ventilator control unit 21 gets disconnected from the actuator, and user adjustable control, for example an adjustment knob 14, of the another electrical control system drives the exhalation valve coil actuator 19 instead providing user-adjustable APL control that is independent of the ventilator electronics. Figure 3 shows separate selector switch knob 13 and adjustment knob 14 but these can naturally be combined into a same knob. All kinds of control knobs can naturally be used. Alternative implementation for a user interface 20 can be used, or the APL control mode can be activated by automatic ventilator failure detection logic. For this purpose the selector switch 24 must have an electrically accessible control mechanism.

The capability for using exhalation valve for APL operation independently of ventilator 1 function is arranged by another electrical control system 12, 14, 16 as shown in Figure 3.

The electric current controlled APL mode can be activated either by the user turning or moving the selector switch knob 13 of the selector switch 24, or alternatively or additionally by an automatic safety mechanism that activates the current-controlled APL mode in case of loss of ventilator functionality for whatever reason, for example failure in ventilator itself or loss of ventilator power supply.

Because the force exerted by the electromagnetic actuator 10 towards the flow control element 9 depends on the coil current, user-controllable current source, i.e. the adjustment knob 14 connected to exhalation valve can provide APL operation corresponding traditional spring-loaded mechanical APL valve arrangement. The dependency between the force exerted by the electromagnetic actuator 10 towards the flow control element and the coil current may be for example linear. It is however quite possible to use any other appropriate dependencies.

To assist user in setting appropriate APL levels, a pressure scale 15 corresponding to APL controller positions can be shown either as a calibrated scale drawn near the control knob 14 if such is used, or on an electrically controlled display that shows pressure readings corresponding to current being fed into the valve coil. The electrically controlled display may be for example a digitally controlled display, an analog indicator or any other appropriate device.

To allow using the described APL control mode in cases that the ventilator functionality is lost due to loss of system main power supply, the presented another electrical current control system 12, 14, 16 shall allow taking its power from such independent backup power source 16 that is available even in case of main power failures. The independent power source can be for example dedicated batteries or system backup batteries.

Although ventilator failures are most likely due to other reasons than failures in the exhalation valve actuator coil component itself, it is not impossible that also the exhalation valve actuator coil might fail also, for example by burning i.e. fusing due to coil material failure or unintended current overloading. This issue can be mitigated by using an actuator with dual coil winding 25, 26 instead of single winding 17 shown in Figure 2. In such arrangement the ventilator can control the exhalation valve actuator by feeding electric current into the primary coil winding, and in case of ventilator or first coil winding failure, the presented control system can apply its control current into a second coil 26 winding which works even in case that a primary coil 25 were fused.

This written description uses examples to disclose the invention, including the best mode, and also to enable any person skilled in the art to practice the invention, including making using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Breathing circuit pressure control system in connection of ventilation, controlling pressure in an airway line of the breathing circuit, the breathing circuit pressure control system comprising,
a flow control element (9) for controlling flow resistance in the airway line (11),
an electromagnetic actuator (10) for creating a force acting on the flow control element (9), and
a ventilator control unit (21) to control the electromagnetic actuator for creating a force acting on the flow control element (9),
**characterized in that**
the breathing circuit pressure control system further comprises an another electrical control system (12,14,16) to control the electromagnetic actuator for creating a force acting on the flow control element (9), and
a selector switch (24) for selecting the another electrical control system arranged to control the electromagnetic actuator and disconnect the ventilator control unit (21) from controlling the electromagnetic actuator (10).

2. Breathing circuit pressure control system as claimed in claim 1, **characterized in that** the selector switch (24) for selecting the another electrical control unit is manually accessible for the user.

3. Breathing circuit pressure control system as claimed in claim 1, **characterized in that** the another electrical control system (12,14,16) is provided with an automatic safety mechanism arranged to activate the another electrical control system.

4. Breathing circuit pressure control system as claimed in claim 1, **characterized in that** the another electrical control system (12,14,16) comprises a user adjustable electric control.

5. Breathing circuit pressure control system as claimed in claim 4, **characterized in that** the user adjustable electric control is an adjustment knob (14).

6. Breathing circuit pressure control system as claimed in claim 1, **characterized in that** the electromagnetic actuator (10) comprises at least one coil (25,26) and that the another electrical control system (12,14,16) is arranged to control the coil current.

7. Breathing circuit pressure control system as claimed in claim 6, **characterized in that** the force created by the electromagnetic actuator (10) towards flow control element (9) is arranged to depend on the coil current.

8. Breathing circuit pressure control system as claimed in claims 2 and 5, **characterized in that** the selector switch knob (13) and the adjustment knob (14) has been combined into the same knob.

9. Breathing circuit pressure control system as claimed in claim 7, **characterized in that** a pressure scale (15) corresponding to the knob position is provided near the adjustment knob (14).

10. Breathing circuit pressure control system as claimed in claim 9, **characterized in that** the pressure scale (15) is a calibrated scale showing pressure readings corresponding to the current fed into the coil.

11. Breathing circuit pressure control system as claimed in claim 10, **characterized in that** the pressure scale (15) is an electrically controlled display showing pressure readings corresponding to current fed into the coil.

12. Breathing circuit pressure control system as claimed in claim 1, **characterized in that** the another electrical control system (12,14,16) is provided with an independent power source (16).

13. Breathing circuit pressure control system as claimed in claim 6, **characterized in that** the electromagnetic actuator (10) comprises a dual coil winding construction (25, 26).

14. Breathing circuit pressure control system as claimed in claim 1, **characterized in that** the system comprises a mechanical ventilation system and a manual ventilation system.

15. Breathing circuit pressure control system as claimed in claim 14 **characterized in that** the another electrical control system (12,14,16) has been arranged to be used when the manual ventilation system is used.
